# EUROPÄISCHE PATENTSCHRIFT

(11) **EP 1 038 543 B1**
(45) Veröffentlichungstag und Bekanntmachung des Hinweises auf die Patenterteilung: **27.03.2002**
(21) Anmeldenummer: 99122359.5
(22) Anmeldetag: 10.11.1999
(51) Int. Cl.: A61M 5/315, A61M 5/28

(54) **Spritze für medizinische Zwecke**
Syringe for medical purposes
Seringue pour buts médicaux

(30) Priorität: 19.03.1999 DE 19912322
(43) Veröffentlichungstag der Anmeldung: 27.09.2000
(73) Patentinhaber: Arzneimittel GmbH Apotheker Vetter & Co. Ravensburg, D-88212 Ravensburg (DE)
(72) Erfinder: Vetter Udo, J., D-88214 Ravensburg (DE); Schönwetter Klaus, D-88289 Waldburg (DE)
(74) Vertreter: Dziewior, Joachim, Dipl.-Phys. Dr.

(56) Entgegenhaltungen:
- CH-A- 268 694
- FR-A- 1 412 547
- FR-A- 2 024 089
- US-A- 5 080 649

## Beschreibung

Die Erfindung betrifft eine Spritze für medizinische Zwecke mit einem Spritzenzylinder, wenigstens einem darin angeordneten und mittels einer Kolbenstange verstellbaren Stopfen sowie einer endseitig am Spritzenzylinder angeschlossenen Fingerauflage, die mit einer Durchtrittsöffnung für die Kolbenstange versehen ist, insbesondere in Form einer Doppelkammerspritze, bei der der pharmazeutische Wirkstoff und das Lösungsmittel bis zur Applikation mittels eines weiteren Stopfens voneinander getrennt in einer distalen und einer proximalen Kammer angeordnet sind und über einen by-pass zusammengeführt werden können.

Derartige Spritzen sind in vielfältigen Varianten aus der Praxis bekannt. Dabei ist es insbesondere bei Doppelkammerspritzen entscheidend, den pharmazeutischen Wirkstoff, der in pulverähnlicher Form vorliegt, mit dem Lösungsmittel unmittelbar vor der Anwendung kontaminationsfrei zu vermischen, also zu rekonstituieren, wobei dieser Vorgang möglichst gleichmäßig erfolgen soll, um eine möglichst optimale Rückführung der pharmazeutischen Substanz in den ursprünglichen Zustand zu erreichen. Hierzu wurde auch bereits vorgeschlagen, die Kolbenstange mit einem Gewinde zu versehen, so daß durch die schraubende Bewegung der Kolbenstange ein gleichmäßiger Vortrieb des Kolbens mit reduzierter Geschwindigkeit sichergestellt ist.

Darüber hinaus ist es jedoch oftmals erforderlich, die rekonstituierte Substanz sequentiell, also in mehreren Teilmengen, zu verabreichen, wie dies beispielsweise beim Setzen von Quaddeln in der Neuraltherapie gewünscht ist.

Die Druckschrift FR 2 024 089 A offenbart eine Spritze, welche dem Oberbegriff des Anspruchs 1 entspricht.

Der Erfindung liegt die Aufgabe zugrunde, eine Spritze der eingangs genannten Art so auszubilden, daß zum einen ein kontrolliert ablaufender Rekonstitutionsvorgang gewährleistet ist und darüber hinaus eine sequentielle Verabreichung der rekonstituierten Substanz ermöglicht wird.

Diese Aufgabe wird nach der Erfindung dadurch gelöst, daß die Kolbenstange mit radial vorstehenden Rastelementen versehen ist, für deren Durchtritt durch die Fingerauflage die Durchtrittsöffnung mit wenigstens einer Randaussparung versehen ist, wobei die Rastelemente in axialer Richtung der Kolbenstange zu mehreren hintereinander und/oder axial aufeinanderfolgend wechselweise um einen bestimmten Drehwinkel um die Längsachse der Kolbenstange versetzt angeordnet sind, und wobei im Bereich der Randaussparung ein elastisch federndes Klemmelement vorgesehen ist, das der Kolbenstange bzw. den Rastelementen anliegt.

Der durch die Erfindung erreichte Vorteil besteht im wesentlichen darin, daß zunächst bei der Rekonstituierung der pharmazeutischen Substanz die in axialer Richtung angeordneten Rastelemente an dem bzw. den elastisch federnden Klemmelementen eine Abbremsung insoweit erfahren, daß die Überleitung des Lösungsmittels in die distale, also die kanülenseitige Kammer gegenüber einer glatten Kolbenstange vergleichsweise langsam erfolgt. Die demgegenüber axial aufeinanderfolgend wechselweise um einen bestimmten Drehwinkel angeordneten Rastelemente ermöglichen das sequentielle Injizieren von Teilmengen der Substanz, wobei das jeweils nachfolgende Rastelement stets einen die weiteren Injektion begrenzenden Anschlag bildet. Eine weitere Injektion ist dann jeweils erst nach entsprechendem Drehen der Kolbenstange möglich.

In bevorzugter Ausführungsform der Erfindung sind die Rastelemente zweckmäßigerweise paarweise diametral sich gegenüberstehend angeordnet. Bei den aufeinanderfolgend wechselweise angeordneten Rastelementen empfiehlt es sich, daß der Drehwinkel zwischen aufeinanderfolgenden Rastelementen 90° beträgt.

Um einerseits eine optimale Rekonstituierung des pharmazeutischen Produktes zu erreichen, andererseits zu verhindern, daß hierbei die Kolbenstange etwas zu tief in den Spritzenzylinder eingedrückt wird und hierdurch bereits ein Teil der in der Regel teuren pharmazeutischen Substanz über die Kanüle entweicht, ist im Rahmen der Erfindung vorgesehen, daß die Länge des in ihrer axialen Richtung mit Rastelementen besetzten Teils der Kolbenstange so bemessen ist, daß das Lösungsmittel vollständig in die distale Kammer übergeleitet ist, sobald das letzte der Rastelemente das Klemmelement überwunden hat. Hier schließt sich dann im übrigen in der Regel ein weiteres, bezüglich seines Drehwinkels versetzt angeordnetes Rastelement an, wodurch - vor der sich anschließenden Entlüftung des Spritzenzylinders und der Injektion - zunächst eine weitere Verstellung des Spritzenkolbens verhindert wird.

Für das sequentielle Injizieren des pharmazeutischen Produktes ist es zweckmäßig, wenn der Abstand der unter einem bestimmten Drehwinkel versetzt gegeneinander angeordneten Rastelemente so bemessen ist, daß durch den zwischen zwei Rastelementen möglichen Verstellhub der Kolbenstange eine vorbestimmte Teilmenge injiziert wird. Für die Injizierung unterschiedlicher Teilmengen sind im Rahmen der Erfindung entsprechende Kolbenstangen mit angepaßtem Abstand der Rastelemente vorgesehen. Auf diese Weise läßt sich die für sequentielle Injektionen vorgesehene Teilmenge problemlos durch Verwendung einer bestimmten Kolbenstange vorgeben, wobei die Kolbenstangen in übersichtlicher Weise - beispielsweise durch entsprechende Farbgebung - markiert sein können.

Die Randaussparung der Durchtrittsöffnung in der Fingerauflage besitzt zweckmäßigerweise eine rechtwinklige Form, wobei durchaus auch andere, beispielsweise sektorförmige Gestaltungen denkbar sind.

Das Klemmelement ist vorteilhafterweise lippenartig ausgebildet und hinter der Randaussparung ins Lumen des Spritzenzylinders weisend angeordnet.

Schließlich ist es im Rahmen der Erfindung zweckmäßig, wenn die Rastelemente im Querschnitt quader- oder keilförmige, mit dem Keilwinkel zum Stopfen weisende Gestalt besitzen.

Im folgenden wird die Erfindung an in der Zeichnung dargestellten Ausführungsbeispielen näher erläutert; es zeigen:
- Fig. 1: in den Teilfiguren a-f die schrittweise Vorbereitung einer Doppelkammerspritze für die Injektion,
- Fig. 2: in den Teilfiguren a-e die sequentielle Verabreichung von Teilmengen der im Spritzenzylinder vorhandenen pharmazeutischen Substanz.

Die in der Zeichnung dargestellte Spritze für medizinische Zwecke besteht zunächst aus einem Spritzenzylinder 1, in dem ein üblicher Stopfen 2 angeordnet ist, der über eine Kolbenstange 3 verstellbar ist. Am einen Ende des Spritzenzylinders 1 ist eine Fingerauflage 4 angeschlossen, die mit einer Durchtrittsöffnung 5 für die Kolbenstange 3 versehen ist.

Im Spritzenzylinder 1 ist ein weiterer Stopfen 6 angeordnet, durch den dieser in zwei voneinander getrennte Kammern 7,8 unterteilt ist. Der pharmazeutische Wirkstoff ist dabei in pulverförmiger, in der Regel gefriergetrocknetem Zustand in der distalen Kammer 7 angeordnet, während die proximale Kammer 8 mit dem zugehörigen Lösungsmittel gefüllt ist. Das Lösungsmittel kann durch Ausüben von Druck auf die Kolbenstange 3 über einen by-pass 9 in die kanülenseitige, den pharmazeutischen Wirkstoff enthaltende Kammer 7 überführt werden, wobei sich der die beiden Kammern 6,7 voneinander trennende Mittelstopfen 6 in den Bereich des by-passes 9 verlagert.

Um ein zu schnelles Überströmen des Lösungsmittels in die distale Kammer 7 zu verhindern, ist die Kolbenstange 3 mit radial vorstehenden Rastelementen 10 versehen, für deren Durchtritt durch die Fingerauflage 4 Randaussparungen 11 in der Durchtrittsöffnung 5 vorgesehen sind. Die Rastelemente 10 sind in axialer Richtung der Kolbenstange 3 zu mehreren hintereinander angeordnet, wobei im Bereich der Randaussparung 11 ein elastisch federndes Klemmelement 12 vorgesehen ist. Dieses Klemmelement 12 liegt der Kolbenstange 3 bzw. den Rastelementen 10 an und bremst den Vorschub der Kolbenstange 3, so daß eine langsame Durchmischung des Lösungsmittels mit der pharmazeutischen Substanz gewährleistet wird.

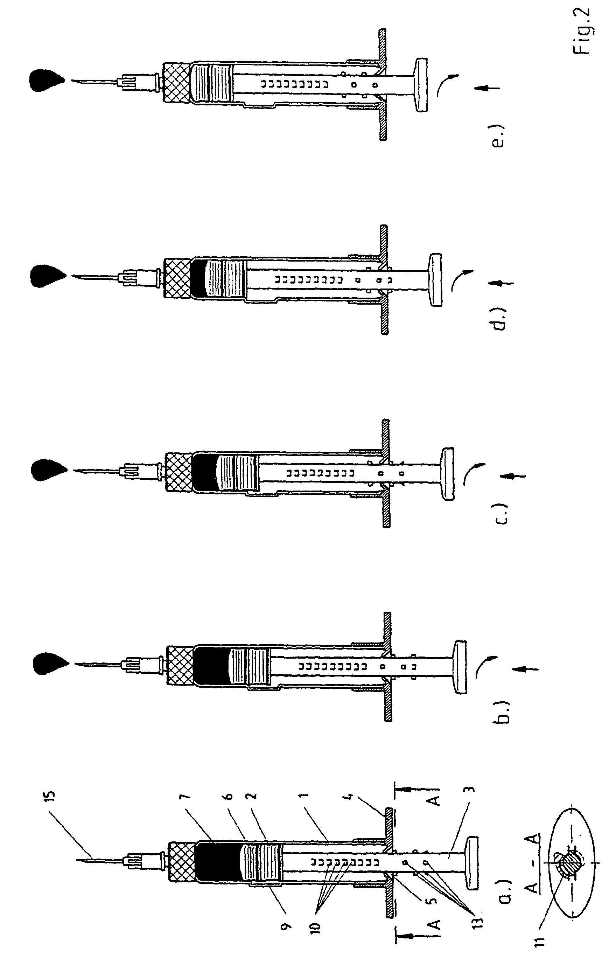

Zusätzlich sind, wie sich insbesondere aus der Fig. 2 ergibt, weitere Rastelemente 13 vorgesehen, die axial aufeinanderfolgend wechselweise um einen bestimmten Drehwinkel um die Längsachse der Kolbenstange 3 versetzt angeordnet sind. Diese Rastelemente 13 bilden jeweils einen Anschlag an der Fingerauflage 4 und dienen dazu, Teilmengen der im Spritzenzylinder 1 enthaltenen Substanz sequentiell zu injizieren. Jedesmal, wenn eines der Rastelemente 13 an der Fingerauflage 4 anschlägt, muß die Kolbenstange 3 um einen durch die Anordnung der Rastelemente 13 vorgegebenen Drehwinkel gedreht werden, so daß dann das eben noch als Anschlag dienende Rastelement 13 durch die Randaussparung 11 durchtreten kann und somit die Injektion einer weiteren Teilmenge ermöglicht, bis das nächstfolgende Rastelement 13 erneut an der Fingerauflage 4 anschlägt.

Die Rastelemente 10,13 sind, wie sich aus der Zeichnung ersehen läßt, paarweise diametral sich gegenüberstehend angeordnet. Weiter beträgt der Drehwinkel - wie ebenfalls aus der Zeichnung folgt - zwischen aufeinanderfolgenden Rastelementen 13 jeweils 90°.

Die Länge des Teils der Kolbenstange 3, die in axialer Richtung mit Rastelementen 10 besetzt ist, ist so bemessen, daß das Lösungsmittel vollständig in die distale Kammer 7 übergeleitet ist, sobald das letzte der Rastelemente 10 das Klemmelement 12 überwunden hat. Damit erfolgt eine Bremsung der Kolbenstange 3 während der gesamten Zeit des Überfüllens des Lösungsmittels in die distale Kammer 7 und endet - zweckmäßigerweise durch Anschlag eines demgegenüber gedrehten Rastelementes 13 - genau in dem Augenblick, zu dem sich die beiden Stopfen 2,6 einander anlegen.

Um jeweils vorgegebene Teilmengen der pharmazeutischen Substanz in genau der vorgeschriebenen Dosierung applizieren zu können, ist der Abstand der unter einem bestimmten Drehwinkel versetzt gegeneinander angeordneten Rastelemente 13 so bemessen, daß der sich durch den Abstand zweier benachbarter Rastelemente 13 ergebende Verstellhub der Kolbenstange 3 genau zur Injizierung der gewünschten Teilmenge führt.

Um hierbei auch unterschiedliche Teilmengen injizieren zu können, ist es ausreichend, entsprechend unterschiedliche Kolbenstangen 3 mit angepaßtem Abstand der Rastelemente 13 vorzusehen. Diese Kolbenstangen 3 können zum einen entsprechende Markierungen beispielsweise in Form von Farben besitzen, um möglichst Verwechslungen auszuschließen und dabei entweder einzeln oder als Auswahl der Spritze beigegeben sein.

Die Randaussparung 11 der Durchschnittsöffnung 5 weist in der Regel rechteckige Form auf, kann jedoch auch bogen-bzw. sektorförmig gestaltet sein.

Das Klemmelement 12 ist lippenartig ausgebildet und hinter der Randaussparung 11 in das Lumen des Spritzenzylinders 1 weisend angeordnet.

Wie sich weiter aus der Zeichnung ergibt, besitzen die Rastelemente 10,13 im Querschnitt quader- oder keilförmige Gestalt, wobei im letzteren Fall der Keilwinkel zum Stopfen 2 hin weist.

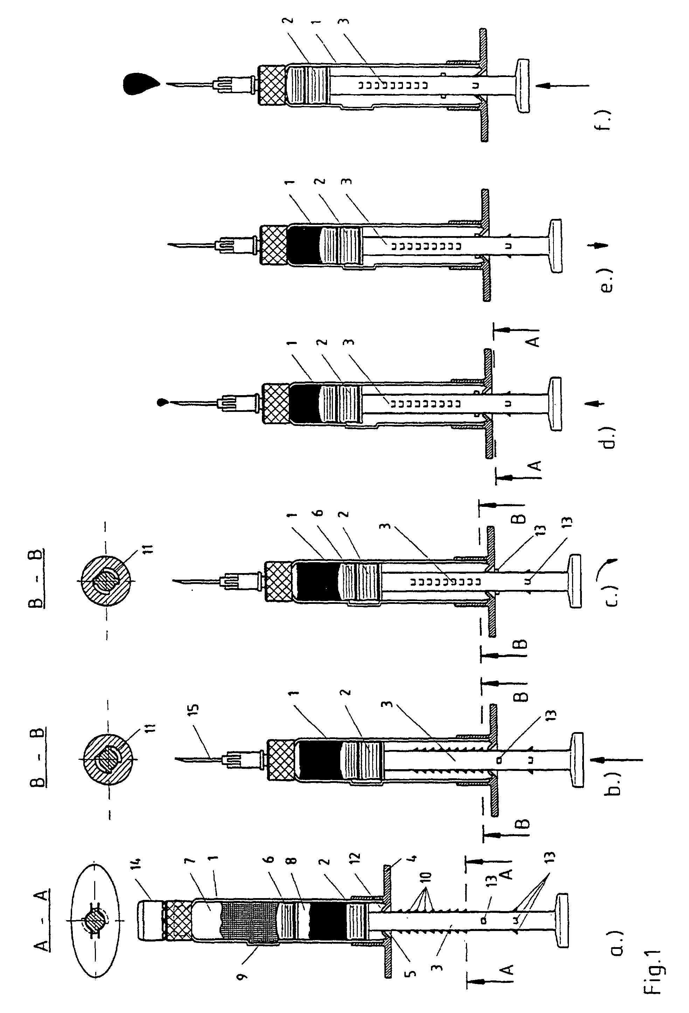

In Fig. 1 ist in der Teilfigur a zunächst die Spritze im Ausgangszustand wiedergegeben, in welchem sie am kanülenseitigen Ende noch mit einem Verschluß 14 versehen ist. In der Teilfigur b ist dann bereits die Kanüle 15 aufgesetzt, wobei im übrigen das Lösungsmittel bereits in die distale Kammer 7 übergeführt ist und die beiden Stopfen 2,6 einander anliegen. In dieser Stellung ist die Kolbenstange 3 an einer weiteren Verstellung gehindert, da ein Rastelement 13 das weitere Vorschieben verhindert. In der Teilfigur c ist die Kolbenstange 3 inzwischen um 90° gedreht, wodurch die eben noch als Anschlag wirkenden Rastelemente 13 durch die Randaussparung 11 ins Innere des Spritzenzylinders 1 eintreten können.

In der Teilfigur d erfolgt die Entlüftung der Spritze; die Teilfigur e deutet den Zustand an, daß nach der Punktion des Gefäßes bei der Injektion zunächst der Spritzenkolben 2 etwas zurückgezogen wird, um zu prüfen, ob Blut am distalen Ende der Spritze erscheint. Sofern dies der Fall ist, kann anschließend die Injektion erfolgen, bis der Spritzenzylinder 1 gemäß Teilfigur f vollständig entleert ist.

Die Fig. 2 geht aus von dem Zustand, wie er in der Fig. 1b wiedergegeben ist und demonstriert in den Teilfiguren b-e, wie durch jeweiliges Drehen der Kolbenstange 3 mehrere, hier insgesamt vier Teilmengen sequentiell injiziert werden können. Nach der Injektion jeder Teilmenge ist eine Drehung der Kolbenstange 3 erforderlich, so daß jedes Rastelement 13, nachdem es zunächst als Anschlag gedient hat, anschließend durch die Randaussparung 11 ins Innere des Spritzenzylinders 1 eintreten kann.

## Patentansprüche

1. Spritze für medizinische Zwecke, mit einem Spritzenzylinder (1), wenigstens einem darin angeordneten und mittels einer Kolbenstange (3) verstellbaren Stopfen (2) sowie einer proximal endseitig am Spritzenzylinder (1) angeschlossenen Fingerauflage (4), die mit einer Durchtrittsöffnung (5) für die Kolbenstange (3) versehen ist, insbesondere in Form einer Doppelkammerspritze, bei der der pharmazeutische Wirkstoff und das Lösungsmittel bis zur Applikation mittels eines weiteren Stopfens (6) voneinander getrennt in einer distalen und einer proximalen Kammer (7,8) angeordnet sind und über einen by-pass (9) zusammengeführt werden können, wobei die Kolbenstange (3) mit radial vorstehenden Rastelementen (10,13) versehen ist, für deren Durchtritt durch die Fingerauflage (4) die Durchtrittsöffnung (5) mit wenigstens einer Randaussparung (11) versehen ist, wobei die Rastelemente (10,13) in axialer Richtung der Kolbenstange (3) zu mehreren hintereinander und/oder axial aufeinanderfolgend wechselweise um einen bestimmten Drehwinkel um die Längsachse der Kolbenstange (3) versetzt angeordnet sind, **dadurch gekennzeichnet, daß** im Bereich der Randaussparung (11) ein elastisch federndes Klemmelement (12) vorgesehen ist, das der Kolbenstange (3) bzw. den Rastelementen (10) anliegt.

2. Spritze nach Anspruch 1, **dadurch gekennzeichnet, daß** die Rastelemente (10,13) paarweise diametral sich gegenüberstehend angeordnet sind.

3. Spritze nach Anspruch 1 oder 2, **dadurch gekennzeichnet, daß** der Drehwinkel zwischen aufeinanderfolgenden Rastelementen (13) 90° beträgt.

4. Spritze nach einem der Ansprüche 1 bis 3, **dadurch gekennzeichnet, daß** die Länge des in ihrer axialen Richtung mit Rastelementen (10) besetzten Teils der Kolbenstange (3) so bemessen ist, daß das Lösungsmittel vollständig in die distale Kammer (7) übergeleitet ist, sobald das letzte der Rastelemente (10) das Klemmelement (12) überwunden hat.

5. Spritze nach einem der Ansprüche 1 bis 3, **dadurch gekennzeichnet, daß** der Abstand der unter einem bestimmten Drehwinkel versetzt gegeneinander angeordneten Rastelemente (13) so bemessen ist, daß durch den zwischen zwei Rastelementen (13) möglichen Verstellhub der Kolbenstange (3) eine vorbestimmte Teilmenge injiziert wird.

6. Spritze nach Anspruch 5, **dadurch gekennzeichnet, daß** für die Injizierung unterschiedlicher Teilmengen entsprechende Kolbenstangen (3) mit angepaßtem Abstand der Rastelemente (13) vorgesehen sind.

7. Spritze nach einem der Ansprüche 1 bis 6, **dadurch gekennzeichnet, daß** die Randaussparung (11) der Durchtrittsöffnung (5) rechtwinklige Form besitzt.

8. Spritze nach einem der Ansprüche 1 bis 6, **dadurch gekennzeichnet, daß** die Randaussparung (11) der Durchtrittsöffnung (5) bogen- bzw. sektorförmige Gestalt besitzt.

9. Spritze nach einem der Ansprüche 1 bis 8, **dadurch gekennzeichnet, daß** das Klemmelement (12) lippenartig ausgebildet und hinter der Randaussparung (11) ins Lumen des Spritzenzylinders (1) weisend angeordnet ist.

10. Spritze nach einem der Ansprüche 1 bis 9, **dadurch gekennzeichnet, daß** die Rastelemente (10,13) im Querschnitt quader- oder keilförmige, mit dem Keilwinkel zum Stopfen (2) weisende Gestalt besitzen.

## Claims

1. Syringe for medical purposes, with a syringe barrel (1), at least one plug (2) which is located inside it and is adjustable by means of a piston rod (3), as well as a finger rest (4) which is connected to the proximal end of the syringe barrel (1) and is equipped with a through-opening (5) for the piston rod (3), in particular in the form of a dual chamber syringe, in which up until application the pharmaceutical active substance and the solvent are arranged in a distal and a proximal chamber (7, 8) separated from one another by means of a further plug (6) and are brought together via a by-pass (9),
wherein the piston rod (3) is equipped with radially projecting catch elements (10, 13), for whose emergence through the finger rest (4), the through-opening (5) is equipped with at least one edge recess (11), wherein the catch elements (10, 13) are arranged in an axial direction of the piston rod (3), offset at a particular rotational angle around the longitudinal axis of the piston rod (3), to form multiples one after another and/or following one another axially alternately,
**characterised in that**
provided in the area of the edge recess (11) is an elastically sprung jamming element (12) which rests against the piston rod (3) or the catch elements (10).

2. Syringe in accordance with claim 1, **characterised in that** the catch elements (10, 13) are arranged in pairs, diametrically opposite one another.

3. Syringe in accordance with claim 1 or 2, **characterised in that** the rotational angle between catch elements (13) following on from one another is 90°.

4. Syringe in accordance with one of the claims 1 to 3, **characterised in that** the length of that part of the piston rod (3) which is equipped in its axial direction with catch elements (10) is dimensioned such that the solvent is completely transferred into the distal chamber (7) as soon as the last of the catch elements (10) has surmounted the jamming element (12).

5. Syringe in accordance with one of the claims 1 to 3, **characterised in that** the distance between the catch elements (13), which are arranged offset relative to one another by a certain rotational angle, is dimensioned such that through the adjustment stroke of the piston rod (3) which is possible between two catch elements (13), a predetermined partial amount is injected.

6. Syringe in accordance with claim 5, **characterised in that** for the different partial amounts for injection, corresponding piston rods (3) with adjusted distances between the catch elements (13) are provided.

7. Syringe in accordance with one of the claims 1 to 6, **characterised in that** the edge recess (11) of the through-opening (5) has a rectangular form.

8. Syringe in accordance with one of the claims 1 to 6, **characterised in that** the edge recess (11) of the through-opening (5) has an arc shape or sector shape.

9. Syringe in accordance with one of the claims 1 to 8, **characterised in that** the jamming element (12) is designed in a lip-like manner and is arranged behind the edge recess (11), pointing into the lumen of the syringe barrel (1).

10. Syringe in accordance with one of the claims 1 to 9, **characterised in that** the catch elements (10, 13) have a cross section which is cuboid or wedge-shaped, with the wedge angle pointing towards the plug (2).

## Revendications

1. Seringue à usage médical, comprenant un corps de seringue (1), au moins un piston (2) mobile à l'intérieur de celui-ci, qui peut être déplacé à l'aide d'une tige de piston (3), ainsi qu'une collerette repose-doigts (4) liée à l'extrémité proximale du corps de seringue, qui est pourvue d'une ouverture de passage (5) pour la tige de piston (3), en particulier seringue à double chambre, dans laquelle le principe actif pharmaceutique et le solvant, jusqu'à l'application, sont disposés dans une chambre distale et une chambre proximale (7, 8), en étant séparés l'un de l'autre par un piston (6) supplémentaire, et peuvent être mélangés par l'intermédiaire d'un by-pass, la tige de piston (3) étant munie de crans (10, 13) saillants dans la direction radiale, pour le passage desquels la collerette repose-doigts (4) est pourvue d'au moins une échancrure (11), les crans (10, 13), dans la direction axiale de la tige de piston (3), étant disposés à plusieurs l'un derrière l'autre et/ou les uns à la suite des autres dans la direction axiale en étant décalés d'un certain angle autour de l'axe de rotation de la tige de piston (3), **caractérisée en ce qu'**il est prévu dans la région de l'échancrure (11), un élément de retenue (12) élastique qui est appliqué contre la tige de piston (3) ou les crans (10).

2. Seringue selon la revendication 1, **caractérisée en ce que** les crans (10, 13) sont disposés par paire, en étant diamétralement opposés.

3. Seringue selon la revendication 1 ou 2, **caractérisée en ce que** l'angle de rotation entre des crans consécutifs (13) est de 90°.

4. Seringue selon une des revendications 1 à 3, **caractérisée en ce que** la longueur de la partie de la tige de piston pourvue de crans (10) dans sa direction axiale est telle, que le solvant soit entièrement transféré dans la chambre distale (7) au moment où le dernier cran (10) a passé l'élément de retenue (12).

5. Seringue selon une des revendications 1 à 3, **caractérisée en ce que** la distance entre les crans (13) mutuellement décalés d'un angle de rotation donné est telle, qu'un volume partiel prédéterminé soit injecté avec la course de déplacement permise entre deux crans (13).

6. Seringue selon la revendication 5, **caractérisée en ce que** pour l'injection de volumes partiels différents, il est prévu des tiges de piston (3) adaptées, avec un espacement adapté des crans (13)

7. Seringue selon une des revendications 1 à 6, **caractérisée en ce que** l'échancrure (11) dans l'ouverture de passage (5) a une forme rectangulaire.

8. Seringue selon une des revendications 1 à 6, **caractérisée en ce que** l'échancrure (11) dans l'ouverture de passage (5) a une forme d'arc ou de secteur de cercle.

9. Seringue selon une des revendications 1 à 8, **caractérisée en ce que** l'élément de serrage (12) est conformé en lèvre et est disposé derrière l'échancrure (11) en étant orienté en direction de la lumière du corps de seringue (1).

10. Seringue selon une des revendications 1 à 9, **caractérisée en ce que** les crans (10, 13), en section transversale, ont une forme de parallélépipède ou de coin, avec l'angle de coin orienté en direction du piston (2).
